# EUROPEAN PATENT APPLICATION

(11) **EP 3 719 711 A2**
(43) Date of publication of application: **07.10.2020**
(21) Application number: 20188779.1
(22) Date of filing: 30.07.2020
(51) Int. Cl.: G06N 3/04, G06N 3/08

(54) **METHOD OF DETECTING ANOMALOUS DATA, MACHINE COMPUTING UNIT, COMPUTER PROGRAM**

(71) Applicant: Institutul Roman De Stiinta Si Tehnologie, 400504 Cluj Napoca (RO)
(72) Inventor: Malago, Luigi, 400370 Cluj Napoca, Cluj (RO); Albu, Alexandra-Ioana, 400423 Cluj-Napoca, Cluj (RO); Enescu, Alina, 105300 Boldesti-Scaeni, Prahova (RO)
(74) Representative: Vasilescu, Raluca

(57) **Abstract**

This patent refers to a computer-implemented method for detecting anomalous data in a test dataset using two Variational AutoEncoders VAEs by means of using a machine-computing unit, the method comprising training and validation steps as well as testing steps.

In the training and validation steps a first Variational AutoEncoder VAE model (VAE-H) is trained on a first plurality of data cases from a first dataset H corresponding to non-anomalous data cases, whereas a second Variational AutoEncoder VAE model (VAE-U) is trained on a second plurality of data cases from a second dataset U as a mix of unlabelled data with and without anomalies, such that it learns variegated representations of data cases with and without anomalies.

A first plurality of dissimilarity measures is computed between a first encoding of the data case through the second Variational AutoEncoder model (VAE-U) and a second encoding of the reconstructed data case through the second Variational AutoEncoder model (VAE-U) for the data cases of the first plurality, then a first anomaly threshold is computed based on said first plurality of dissimilarity measures.

In the testing steps, the anomalous data is detected in the test dataset comprising at least one raw data case by computing a dissimilarity measure between the first encoding of the raw data case through the second Variational AutoEncoder model (VAE-U) and the second encoding of the reconstruction of the raw data case through the second Variational AutoEncoder model (VAE-U) and comparing the dissimilarity measure with the first anomaly threshold. If the dissimilarity measure is greater than the first anomaly threshold the raw data case is labelled as anomalous and an anomaly mask is determined for localizing the anomaly in the data case. The anomalous data cases and their localization are made accessible to a decision maker.

The patent also discloses a machine computing unit for carrying out the method, a computer program to be executed by the machine computing unit, a computer readable medium and a data stream.

BIBLIOGRAPHICAL REFERENCES

1. Hongchao Song, Zhuqing Jiang, Aidong Men and Bo Yang. A Hybrid Semi-Supervised: Anomaly Detection Model for High-Dimensional Data. Computational Intelligence and Neuroscience Volume 2017, Article ID 8501683, https://doi.org/10.1155/2017/8501683 15 November 2017
2. Li, Xiao-Li, and Bing Liu. Learning from positive and unlabeled examples with different data distributions. In European conference on machine learning, pp. 218-229. Springer, Berlin, Heidelberg, 2005
3. A. Myronenko. 3d mri brain tumor segmentation using autoencoder regularization. In International MICCAI Brainlesion Workshop, pages 311-320. Springer, 2018
4. T. Tashiro, T. Matsubara, and K. Uehara. Deep neural generative model for fmri image based diagnosis of mental disorder. In *Interna-tional Symposium on Nonlinear Theory and its Applications (NOLTA),* 2017
5. Baur, Christoph, et al. "Deep autoencoding models for unsupervised anomaly segmentation in brain MR images." International MICCAI Brainlesion Workshop. Springer, Cham, 2018
6. X. Chen and E. Konukoglu. Unsupervised detection of lesions in brain MRI using constrained adversarial auto-encoders. Medical Imaging for Deep Learning (MIDL 2018), 2018
7. X. Chen, N. Pawlowski, M. Rajchl, B. Glocker, and E. Konukoglu. Deep generative models in the real-world: an open challenge from medical imaging. arXiv:1806.05452, 2018
8. João Pereira, Margarida Silveira. Unsupervised Representation Learning and Anomaly Detection in ECG Sequences. Int. Data Mining and Bioinformatics Vol. x, No. x. 1-18. Available online as of 1 August 2019
9. D. C. Van Essen, K. Ugurbil, E. Auerbach, D. Barch, T. Behrens, R. Bucholz, A. Chang, L. Chen, M. Corbetta, S. W. Curtiss, et al. The human connectome project: a data acquisi- tion perspective. *Neuroimage,* 62(4):2222-2231, 2012
10. B. H. Menze, A. Jakab, S. Bauer, J. Kalpathy- Cramer, K. Farahani, J. Kirby, Y. Burren, N. Porz, J. Slotboom, R. Wiest, et al. The multimodal brain tumor image segmentation benchmark (BRATS). *IEEE transactions on medical imaging,* 34(10):1993-2024, 2014
11. M. Kistler, S. Bonaretti, M. Pfahrer, R. Niklaus, and P. Bu"chler. The virtual skeleton database: an open access repository for biomedical research and collaboration. *Journal of medical Internet research*, 15(11):e245, 2013
12. Zimmerer, D., Kohl, S.A., Petersen, J., Isensee, F. and Maier-Hein, K.H.. Context-encoding variational autoencoder for unsupervised anomaly detection. Medical Imaging with Deep Learning 2019

## Description

### Field of the invention

The invention is related to the field of detecting anomalous data in a test dataset comprising at least one raw data case using semi-supervised anomaly detection methods. In some embodiments, the data cases are images and in some particular cases the images are used for anomaly detection in medical imaging.

### Terms used in this invention

Throughout this invention, the terms "anomaly", "anomalous" or "outlier" designate data cases among a plurality of data cases of a test dataset that are dissimilar to other data cases of the plurality according to specific criteria. In contrast, the test dataset contains "normal" or, alternatively called, "non-anomalous" data according to the same specific criteria.

For example, in medical imaging, the terms "anomaly", "anomalous" or "outlier" correspond to images corresponding to unhealthy persons, that is, individuals that suffer from various illnesses. In contrast with this, "normal" images refer to images used in medical imaging that correspond to healthy persons. The terms "healthy" and, respectively, "unhealthy" refer to the field of medicine for which the medical imaging is used by the health practitioners. For example, if brain Magnetic Resonance Image MRI is used by the health practitioner to determine whether an individual has a brain lesion or tumour, "healthy" and, respectively, "unhealthy" refer only to the brain as subject of the investigation.

In another example when data cases are sounds produced by the engine of a road vehicle, "normal", "non-anomalous" sounds of the engine are defined in specific terms by the road vehicle manufacturer to refer to the situations when the engine functions properly. The terms "anomaly", "anomalous" or "outlier" refer to sounds that indicate that the engine is not functioning properly and thus intervention of the mechanic is required.

In another example when data cases are sounds associated with the heartbeat of a human or animal, "normal", "non-anomalous" sounds correspond to the healthy human or animal, whereas "anomaly", "anomalous" or "outlier" refer to sounds of a ill heart of the human, respectively animal.

The term "Euclidean distance" has its usual meaning of the ordinary, straight-line distance between two points in the Euclidean geometry.

The term "geometric distance" alternatively called "non-Euclidean distance" has the meaning of the distance between two points computed with respect to a non-Euclidean geometry.

The term "dissimilarity measure", or alternatively called in short "dissimilarity" is used throughout the invention to define the notion of difference in a more general way than the term of distance, be it Euclidean or geometric. Dissimilarity refers to the difference in general. In an example, a person with blue eyes has a dissimilarity in respect to a person with green eyes. Mathematically speaking, dissimilarity measure is less restrictive than distance because it is not necessary that dissimilarity to satisfy all the conditions required to be called a distance, in particular it is not required that the dissimilarity measure be symmetric and verify the triangle inequality.

A divergence is an example of a dissimilarity measure.

### Background of the invention

Computer-implemented processing of test datasets irrespectively of the field of technology is carried out in order to enable a decision maker to take a good decision. In this respect, the decision maker often needs to use automatic analysis to separate normal, non-anomalous data cases from anomalous data cases.

When using medical imaging techniques, health practitioners, as decision makers, spend a large portion of their time in order to read and interpret a large number of images after an imaging protocol has been completed. Non-limiting examples are Magnetic Resonance Images MRIs or Computed Tomography images CTIS imaging protocols that produce a large number of images. The purpose of reading and interpretation of the images is to detect anomalies corresponding to specific illnesses, such as, but not limited to, tumours and lesions.

Therefore, automatic analysis of the medical images is of great help and relevance for the health practitioners, because it helps to reduce the time for analysing each image, and it helps to identify which of the medical images are outliers.

Deep learning technologies are among the techniques used for automatic analysis of large amounts of data, including medical images.

A good summary of anomaly detection methods is given by Hongchao Song et al. in an article published on 15 November 2017 [1]. According to the article, "anomaly detection can be generalized as constructing a model from the given training data and predicting the status of unknown data". Further on, according to the article "traditional distance-based anomaly detection methods compute the neighbourhood distance between each observation and suffer from the curse of dimensionality in high-dimensional space; for example, the distances between any pair of samples are similar and each sample may perform like an outlier".

One of the major difficulties of using supervised deep learning technologies is that annotations for large amounts of data are difficult to be collected. For this reason, unsupervised or semi-supervised anomaly detection techniques are thought to be of use for automatic analysis of the medical images.

The Hongchao Song et al. article gives the main features of unsupervised and semi-supervised anomaly detection methods: "in semi-supervised approaches only normal samples are available in the training set; that is, the user cannot obtain information about anomalies. Unknown samples are classified as outliers when their behaviour is far from that of the known normal samples", whereas "in unsupervised approaches the class information of all samples in the training data is unknown to the researchers; that is, the samples in the training set may contain both normal and anomalous samples, but the classification of each sample is unknown". However, in the presence of unlabelled anomalous samples, there are anomaly detection methods that belong to the category of learning from positive and unlabelled examples [2], which is a specific type of semi-supervised learning.

There are various types of methods of anomaly detection such as classification-based methods, reconstruction-based methods or density-based methods.

Reconstruction-based methods are widely used for medical imaging since they allow a pixel-wise anomaly detection which helps practitioners to delineate pathological conditions from normal conditions. They aim to reconstruct accurately normal data samples, while in the case of anomalous data samples they generate significant reconstruction errors.

Many reconstruction-based methods use Variational AutoEnconders VAEs because they are flexible generative models which can be used for inference on complex datasets. In the literature there are several applications of Variational AutoEnconders VAEs in the medical field such as: segmentation of tumours in brain Magnetic Resonance Images MRI scans [3] or identification of mental disorders such as schizophrenia [4]. VAEs have been successfully used in numerous anomaly detection tasks, for instance to outline tumoral areas or other lesions in brain scans. The majority of these approaches [5,6,7] are reconstruction-based, i.e., they detect anomalous pixels by computing the Euclidean distance between the original image and its reconstruction, said Euclidean distance further called L2 in the input space. Therefore, the Euclidean distances are computed in the input space, which is defined as the coordinate space of the high-dimensional input data, which in case of images is represented by the pixels of the images.

Other methods, such as the one proposed by João Pereira et al. [8], use geometric distances computed in the latent space of a Variational AutoEnconder VAE. João Pereira et al. [8] propose using a VAE parameterized by recurrent neural networks in an approach using unsupervised representation learning of Electrocardiograms ECGs sequences for the task of anomaly detection. The anomaly detection in the ECGs sequences is carried out by computing the Wasserstein distance - which is a geometric distance, between the latent representation of the test data point and the latent representations of a significant set of other input data points. They rely on the assumption that the majority of data samples from a single dataset are not anomalous.

### Disadvantages of prior art

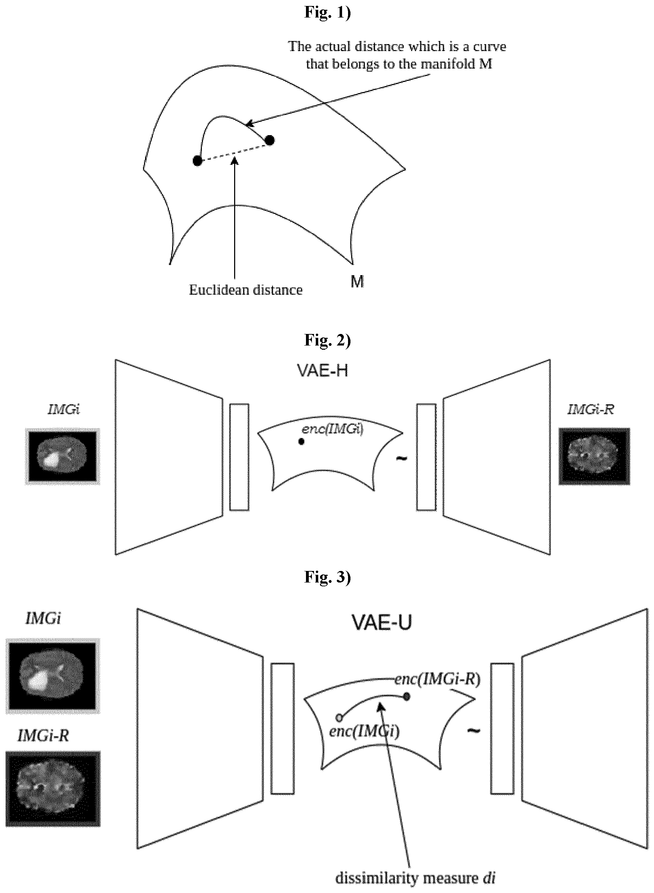

Firstly, computing the Euclidean distance in the input space does not take into account the fact that real-world data cases belong to a restricted subspace of the input space and therefore the manifold structure is ignored in the computation. By computing the distance with respect to the Euclidean ambient space, the constraints set by the distribution of data cases in the input space are bypassed and distances are obtained which correspond to curves which are not forced to belong to the manifold itself. This idea is illustrated in Fig.1), where computing the Euclidean distance between points does not correspond to the actual distance associated to a length-minimizing curve along the manifold itself. As illustrated in Fig. 1), in a manifold, two points may appear close if the Euclidean distance is computed, represented with the dotted line, but because of the manifold structure the actual distance is greater, since it is associated to the length of a curve which belongs to the manifold itself.

Secondly, the computation of the Euclidean distance in the latent space of a VAE depends on the parameterization: if the approximate posterior is parameterized in a different way, for instance through the variances of independent Gaussian variables rather than through the standard deviations, or alternatively, through the precisions - which correspond to the inverse of the variances, then the Euclidean distance between the same distributions would be different.

Thirdly, in some cases, such as when the data cases are images that are used to evaluate the health of the brain structure, the variability of healthy structures is usually greater than the variability given by an anomaly, as noted by Chen et al. [6] so the anomalous points are most likely not separated by non-anomalous ones in the latent space.

### Problem solved by the invention

The problem to be solved is to define a semi-supervised method for anomaly detection, which is able to provide a higher performance of the anomaly detection.

Throughout this invention, the term "higher performance" means that at least one among the following three performance measures: i) accuracy, ii) F1-score, and iii) Area under the Receiver operating characteristic curve ROC AUC has higher values when applying the method of the invention, than when applying other methods of prior art.
i) In the context of machine learning, accuracy is a performance measure used in classification algorithms, alternatively called classifiers. Accuracy is defined as the number of correctly classified samples over the total number of samples. It is known that accuracy alone is not enough to evaluate the performance of classification algorithms.
ii) F1-score is another performance measure of classification algorithms, particularly useful for imbalanced datasets. F1-score is defined as the harmonic mean between precision and recall.
   - Precision is the number of correctly classified positive samples over the number of positive predicted samples.
   - Recall, or alternatively called true positive rate, is defined as the ratio between the number of correctly classified positive samples over the total number of positive samples.
iii) The Area under the Receiver operating characteristic curve ROC AUC is another performance measure of the classifiers based on setting various thresholds for the classification scores. Given some classification scores - that in case of anomaly detection are anomaly scores, the Area under the Receiver operating characteristic curve ROC AUC evaluates the true positive rates and false positive rates for a number of thresholds and computes the area under the obtained curve.

### Summary of the invention

In order to solve the problem, the inventors conceived in a **first** aspect of the invention a computer-implemented method for detecting anomalous data in a test dataset comprising at least one raw data case *IMGi* using two Variational AutoEncoders VAEs, by means of using a machine-computing unit, the method comprising training and validation steps TR and testing steps TS.

### TRAINING AND VALIDATION STEPS:

TR-S1 Collecting simultaneously, by means of a data collecting and pre-processing module, two datasets:
   - a first dataset H containing a first plurality of data cases *IMG-Hi* without anomalies, corresponding to non-anomalous data cases, said first plurality of data cases *IMG-Hi* belonging to a training sub-set of the first dataset H and to a validation sub-set of the first dataset H,
   - a second dataset U containing a second plurality of data cases *IMG-Ui* as a mix of unlabelled data with and without anomalies, corresponding to anomalous data cases and, respectively, non-anomalous data cases, said second plurality of data *cases IMG-Ui* belonging to a training sub-set of the second dataset and to a validation-sub-set of the second dataset U.
TR-S2 Pre-processing by means of the data collecting and pre-processing module said first dataset H and said second dataset U,
TR-S3 Training and validation simultaneously by means of a computing module of:
   - a first Variational AutoEncoder VAE model (VAE-H) on the first plurality of data cases IMG-Hi such that it learns representations of non-anomalous data cases,
   - a second Variational AutoEncoder VAE model (VAE-U) on the second plurality of data cases IMG-Ui such that it learns variegated representations of data cases with and without anomalies.
TR-S4 Carrying out, by means of the computing module, for each data case *IMG-Hi* from the validation sub-set of the first dataset H:
   - determining a reconstruction of the data case *IMG-Hi* using the first Variational AutoEncoder VAE model (VAE-H), resulting the reconstructed data case *IMG-Hi-R,*
   - encoding the data case *IMG-Hi* through the second Variational AutoEncoder model (VAE-U), resulting a first encoding enc*(IMG-Hi),*
   - encoding the reconstructed data case *IMG-Hi-R* through the second Variational AutoEncoder model (VAE-U), resulting a second encoding *enc(IMG-Hi-R),*
TR-S5 Computing, by means of the computing module, of a first dissimilarity measure *d-Hi* between the first encoding enc*(IMG-Hi)* and the second encoding *enc(IMG-Hi-R),* resulting a first plurality of dissimilarity measures corresponding to the first plurality of data cases *IMG-Hi,*
TR-S6 Computing, by means of the computing module, of a data case anomaly threshold *THRj* based on the first plurality of dissimilarity measures computed for the first dataset H,
**TESTING STEPS** for detecting anomalous data in the test dataset comprising the at least one raw data case *IMGi,* said at least one raw data case *IMGi* comprising at least one data point *p*
TS-S1 Receiving, by means of the collecting and pre-processing module, of the at least one raw data case *IMGi* from at least one apparatus providing raw data cases *IMGi* and pre-processing the at least one raw data case *IMGi,*
TS-S2 Carrying out, by means of the computing module,
   - determining a reconstruction of the at least one raw data case *IMGi* using the first Variational AutoEncoder VAE model (VAE-H), resulting the reconstructed data case *IMGi-R*,
   - encoding the at least one raw data case *IMGi* through the second Variational AutoEncoder model (VAE-U), resulting a first encoding *enc(IMGi)*,
   - encoding the reconstructed data case *IMGi-R* through the second Variational AutoEncoder model (VAE-U), resulting a second encoding *enc(IMGi-R),*
TS-S3 Computing, by means of the computing module, of a test dissimilarity measure *di* between the first encoding *enc(IMGi)* and the second encoding *enc(IMGi-R),*
TS-S4 Comparing, by means of the computing module, of said test dissimilarity measure *di* with the data case anomaly threshold *THRj,*
TS-S5 Labelling, by means of a labelling module, of the at least one raw data case *IMGi* as an anomalous data case *IMG-An* if the corresponding test dissimilarity measure *di* computed in TS-S3 is above said data case anomaly threshold THRj,
TS-S6 For the at least one anomalous data case *IMG-An* labelled in TS-S5, localizing, by means of the labelling module, of the anomaly in the at least one anomalous data case *IMG-An,* by determining an anomaly mask of the at least one data point *p,*
TS-S7 Sending, by means of the labelling module, of the anomalous data case *IMG-An* together with the corresponding localization of the anomaly to a communication module accessible to a decision maker

In a **second** aspect of the invention it is provided a machine computing unit comprising at least one processor, at least one memory, a data collecting and pre-processing module, a computing module, a labelling module, and a communication module, the machine computing unit being configured for detecting anomalous data in a test dataset comprising at least one raw data case *IMGi* by means of carrying out the computer-implementing method of the first aspect of the invention in any of its embodiments and combinations thereof.

In a **third** aspect of the invention it is provided a computer program comprising instructions which, when the program is executed by the machine computing unit, cause the machine computing unit to carry out the computer-implementing method of the first aspect of the invention in any of its embodiments and combinations thereof.

In a **fourth** aspect of the invention is provided a computer readable medium having stored thereon instructions which, when executed by the machine computing unit, cause the machine computing unit to carry out the computer-implementing method of the first aspect of the invention in any of its embodiments and combinations thereof.

In a **fifth** aspect of the invention is provided a data stream which is representative of the computer program of the invention.

The invention extends to any novel aspects or features described and/or illustrated herein.

Any feature in one aspect of the invention may be applied to other aspects of the invention, in any appropriate combination. In particular, method features may be applied to machine- computing unit features, and vice versa.

Wherever applicable, means - plus - function features may be expressed alternatively in terms of their corresponding structure, such as a suitably programmed processor and associated memory.

Particular combinations of the various features of the invention can be implemented and/or supplied and/or used independently.

### Advantages of the invention

The main advantages of this invention are the following:
The method according to the invention provides a higher performance of the anomaly detection for the following reasons:
- The method according to the invention carries out a more accurate computation of the test dissimilarity measure *di* between the first latent representation *enc(IMGi)* and the second latent representation *enc(IMGi-R);*
- The method according to the invention is invariant to parametrization, as the computation of the test dissimilarity measure *di* does not depend on the way in which second Variational AutoEncoder VAE model (VAE-U) represents the approximate posterior of said second Variational AutoEncoder VAE model (VAE-U)];
- The method according to the invention addresses all situations, irrespective of whether the variability of the normal data is greater, equal or lower than the variability of the anomalous data, which allows a broader scope of industrial applicability of the invention;
- The method according to the invention is more efficient than certain other methods from prior art since it computes a single test dissimilarity measure *di* as opposed to computing distances for multiple pairs of points, irrespective of whether the distances are Euclidean or geometric.

Yet another category of advantages of the method according to the invention is that, apart from providing higher performance of the detection, the multiple embodiments and combinations thereof allow a flexible application of the invention to a wider range of situations.

### Brief description of the drawings

Fig. 1) An example of a manifold in which the Euclidean distance between two data cases is small, but when the manifold structure is taken into account, the actual distance between the points is larger,
Fig. 2) Schematic illustration of the determination of the reconstructed data cases *IMGi-R* using the first Variational AutoEncoder VAE model (VAE-H) in TS-S2,
Fig 3) Schematic illustration of:
   - encoding the at least one raw data case *IMGi* through the second Variational AutoEncoder model (VAE-U), resulting the first encoding *enc(IMGi)* in TS-S2,
   - encoding the reconstructed data case *IMGi-R* through the second Variational AutoEncoder model (VAE-U), resulting the second encoding *enc(IMGi-R)* in TS-S2,
   - computing of the test dissimilarity measure *di* between the first encoding *enc(IMGi)* and the second encoding *enc(IMGi-R)* in TS-S3,

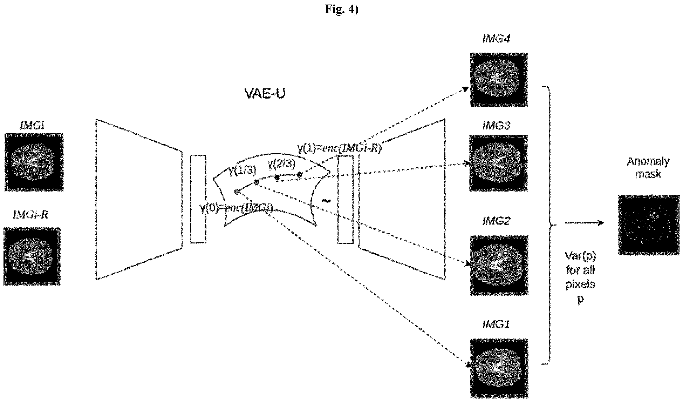

Fig.4) Preferred embodiment showing the determination of the anomaly mask when the data cases are images,

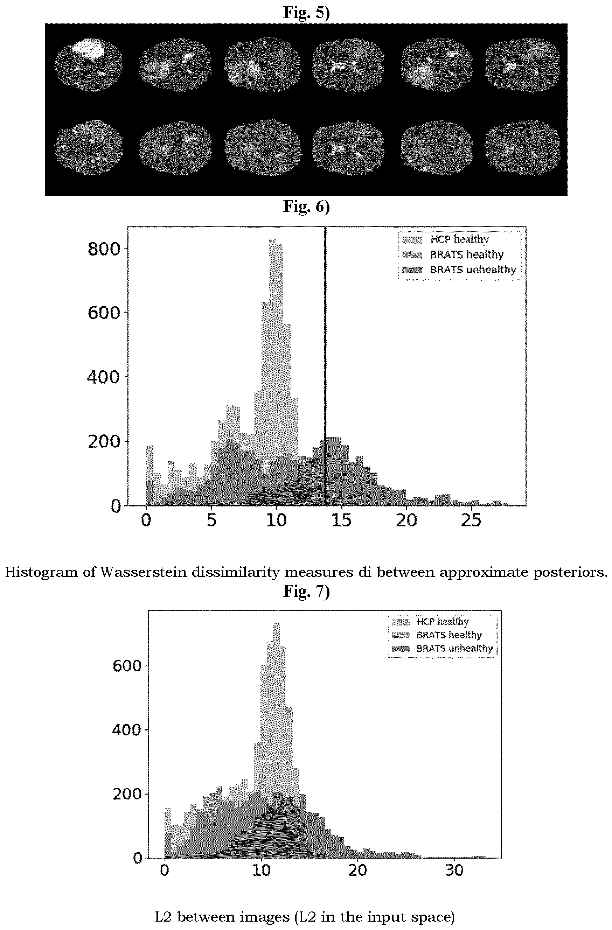

Fig.5) Example of realization: Illustration of reconstructions through VAE-H of images *IMG-Ui,*
Fig.6) Example of realization: Histogram of Wasserstein test dissimilarity measures *di* for the test sub-set of the HCP dataset and the test sub-set of the BRATS-2018 dataset,
Fig. 7) Prior art: Histogram of the L2 Euclidean distance in the input space between the original image and its reconstruction using the first Variational AutoEncoder VAE model (VAE-H) for all the images *IMG-Hi* from the test sub-set of the HCP dataset and respectively all the images *IMG-Ui* the test sub-set of the BRATS-2018 dataset.

### Detailed description

The inventors conceived a computer-implemented method for detecting anomalous data in a test dataset, the test dataset comprising at least one raw data case *IMGi* using two Variational AutoEncoders VAEs, by means of using a machine-computing unit. The method they conceived is applicable to test datasets comprising any type of data such as images, sounds or any other type of signals capable of being transmitted and processed by electronic means, because the machine-learning based method of the invention does not make difference on the nature of data cases.

The method of the invention has training and validation steps, labelled hereafter as TR steps and testing steps, labelled hereafter as TS steps.

In the training steps TR two types of representations are learned:
- representations of non-anomalous data cases, and
- variegated representations of data cases with and without anomalies.

It is known that any dataset used for learning contains training, validation and test sub-sets. Thus:
- the first dataset H contains: a first dataset H training sub-set, a first dataset H validation sub-set and a first dataset H test sub-set,
- the second dataset U contains: a second dataset U training sub-set, a second dataset U validation sub-set and a second dataset U test sub-set,
- The training sub-sets and the validation sub-sets of the first dataset H and, respectively second dataset U are used during the training steps TR, whereas the test sub-sets of the first dataset H and, respectively second dataset U are used during the testing steps TS of the example of realization.

TR-S1 In order to ensure the learning of the two types of representation, two datasets are collected by means of a data collecting and pre-processing module of the machine-computing unit:
- a first dataset H containing a first plurality of data cases *IMG-Hi* without anomalies, corresponding to non-anomalous data cases, said first plurality of data cases *IMG-Hi* belonging to a training sub-set of the first dataset H and to a validation sub-set of the first dataset H,
- a second dataset U containing a second plurality of data cases *IMG-Ui* as a mix of unlabelled data with and without anomalies, corresponding to anomalous data cases and, respectively, non-anomalous data cases, said second plurality of data cases *IMG-Ui* belonging to a training sub-set of the second dataset and to a validation-sub-set of the second dataset U.

Thus both the training sub-set and the validation sub-set of each of the first dataset H and the second dataset U contain data cases from the first plurality of data cases *IMG-Hi* and respectively from the second plurality of data cases *IMG-Ui,*

TR-S2 The data collecting and pre-processing module pre-processes the data of said first dataset H and said second dataset U. Specifically, the data collected and processed is from the respective training and validation sub-sets of the said first dataset H and said second dataset U.

TR-S3 A computing module of the machine-computing unit carries out simultaneous training and validation by means of:
- a first Variational AutoEncoder VAE model (VAE-H) on the first plurality of data cases *IMG-Hi* such that it learns representations of non-anomalous data cases,
- a second Variational AutoEncoder VAE model (VAE-U) on the second plurality of data *cases IMG-Ui* such that it learns variegated representations of data cases with and without anomalies.

Specifically, step TR-S3 is carried out on both respective training and validation sub-sets of the said first dataset H and said second dataset U.

Variational AutoEncoders VAEs are a specific type of autoencoders which have two neural networks. The first neural network of the Variational AutoEncoder VAE is an encoder which maps the input data to the parameters θ of a probability density function qθ over the latent space. The second neural network of the Variational AutoEncoder VAE is the decoder which maps the latent representation to a probability density function pϕ over the space of the observations. Variational AutoEncoders VAEs are trained using principles from variational inference.

In general, for each input x, the Variational AutoEncoder VAE returns an approximate posterior qθ (z|x), from which the latent variables are sampled. This implies that in contrast to a regular AutoEncoder AE, by using Variational AutoEncoders VAEs it is possible to compare the encodings of two different inputs also by computing a dissimilarity function between probability density functions in the space of the approximate posteriors, said dissimilarity function between probability density functions referred to in short throughout the invention as "dissimilarity measure".

Since the inventors aim to provide a higher performance of the anomaly detection as detailed in the section "problem to be solved by the invention", they sought robust and efficient possibilities of computing dissimilarity function between the probability density functions in the space of the latent representations, either the space of the approximate posteriors or its sample space, reason for which they selected Variational AutoEncoders VAEs instead of regular AutoEncoder AEs as a model.

For the same objective to provide a higher performance of the anomaly detection, the inventors sought to use the second dataset U containing the second plurality of data cases *IMG-Ui* as a mix of unlabelled data with and without anomalies in the training and validation steps and not only in the testing steps, as in prior art so that the second Variational AutoEncoder VAE model (VAE-U) learns representations of anomalous data.

TR-S4 The computing module carries out, for each data case *IMG-Hi* of the validation sub-set of the first dataset H the following:
- determines a reconstruction of the data case *IMG-Hi* using the first Variational AutoEncoder VAE model (VAE-H), resulting the reconstructed data case *IMG-Hi-R,*
- encodes the data case *IMG-Hi* through the second Variational AutoEncoder model (VAE-U), resulting a first encoding *enc(IMG-Hi),*
- encodes the reconstructed data case *IMG-Hi-R* through the second Variational AutoEncoder model (VAE-U), resulting a second encoding *enc(IMG-Hi-R),*

The reconstruction of the data case *IMG-Hi-R* is carried out according to prior art. TR-S5 The computing module computes a first dissimilarity measure *d-Hi* between the first encoding *enc(IMG-Hi)* and the second encoding *enc(IMG-Hi-R),* resulting a first plurality of dissimilarity measures corresponding to the first plurality of data cases *IMG-Hi.*

TR-S6 The computing module computes a data case anomaly threshold *THRj* based on the first plurality of dissimilarity measures computed for the first dataset H.

The method according to the invention is conceived to be used in real-life situations when it is required to detect anomalous data in the test dataset comprising one or more raw data cases *IMGi,* said at least one raw data case *IMGi* comprising at least one data point *p*. Thus, in real-life situations, the testing steps refer to said situations when it is required to detect any type of anomalous data: images, sounds or any other type of signals capable of being transmitted and processed by electronic means in technical fields such as, but not limited to: medical field, automotive, phonology computer programmes, speech synthesis, etc. For the purpose of testing the method of the invention, the testing steps TS are carried out on the first dataset H test sub-set and respectively on the second dataset U test sub-set.

For simplicity, when referring hereafter to "one raw data case *IMGi",* it shall be understood the following:
- "one raw data case *IMGi"* stands for one or more raw data cases *IMGi*;
- the "one raw data case *IMGi"* belongs to any of the first dataset H test sub-set and respectively the second dataset U test sub-set, unless explicitly mentioned that it belongs to either the first dataset H test sub-set or the second dataset U test sub-set.

TS-S1 The collecting and pre-processing module receives the raw data case *IMGi* from an apparatus providing raw data cases and pre-processes the raw data case *IMGi.*

TS-S2 With reference to Fig.2) and Fig.3), the computing module carries out the following:
- determines a reconstruction of the at least one raw data case *IMGi* using the first Variational AutoEncoder VAE model (VAE-H), resulting the reconstructed data case *IMGi-R,*
- encodes the raw data case *IMGi* through the second Variational AutoEncoder model (VAE-U), resulting a first encoding enc*(IMGi)*,
- encodes the reconstructed data case *IMGi-R* through the second Variational AutoEncoder model (VAE-U), resulting a second encoding *enc(IMGi-R).*

The reconstruction of the raw data case IMGi-R through VAE-H is exemplified in the example of realization.

The first encoding enc*(IMGi)* is alternatively called the first latent representation *enc(IMGi)*, whereas the second encoding *enc(IMGi-R)* is alternatively called the second latent representation *enc(IMGi-R).* Both latent representations are tensorial representations of the respective raw data cases *IMGi* and *IMGi-R.*

TS-S3 With reference to Fig.3), the computing module computes a test dissimilarity measure *di* between the first encoding enc*(IMGi)* and the second encoding *enc(IMGi-R).*

The computation of the test dissimilarity measure *di* in the space of the approximate posteriors of the second Variational AutoEncoder model (VAE-U) represents a significant departure from the approaches of prior art where various types of dissimilarity measures are computed between the reconstructions in the input space or in the space of the first Variational AutoEncoder (VAE-H). By using in the invention, the test dissimilarity measure *di* - that is invariant to the choice of the parametrization, it is possible to overcome the disadvantage of the dependence on the parametrization of the computation of the Euclidean distance in the latent space.

Even if the method does not need to access any label from the second dataset U, containing data cases with and without anomalies, the method needs to have access to the first dataset H that contains data cases guaranteed to be non-anomalous.

Therefore, the method according to the invention is considered by the inventors to belong to the category of semi-supervised approaches for anomaly detection in particular the method of the invention belongs to the category of positive and unlabelled learning.

TS-S4 The computing module compares said test dissimilarity measure *di* with the data case anomaly threshold *THRj.*

TS-S5 A labelling module labels the raw data case *IMGi* as an anomalous data case *IMG-*An if the corresponding test dissimilarity measure *di* computed in TS-S3 is above said data case anomaly threshold THRj.

It is very useful for the decision makers not only to label anomalous data case *IMG-*An but to localize the anomaly in the data.

TS-S6 For the anomalous data case *IMG-An* labelled in TS-S5, the labelling module localizes the anomaly in the anomalous data case *IMG-An,* by determining an anomaly mask of the at least one data point *p*.

TS-S7 Once the anomalous data is localized, the labelling module sends the anomalous data case *IMG-An* to a communication module accessible to a decision maker. The way the data containing the information that the raw data case *IMG-An* is anomalous together with the corresponding localization of the anomaly is used by the decision maker is outside the scope of this invention.

By training the first Variational AutoEncoder VAE model (VAE-H) and the second Variational AutoEncoder model (VAE-U) to learn compressed low-dimensional representations of the raw data cases in their respective latent spaces and by computing the test dissimilarity measure *di* between the first encoding *enc(IMGi)* and the second encoding *enc(IMGi-R),* the method according to the invention overcomes the disadvantage of ignoring the manifold structure of the space of the approximate posterior when computing Euclidean distances. By comparing the schematic representations of Fig.1) and Fig.3), one can see that the invention has the advantage of a more accurate computation of the test dissimilarity measure *di* between the first latent representation *enc(IMGi)* and the second latent representation *enc(IMGi-R)* which leads to obtaining at least one of the three performance measures, higher for the method, when compared to the L2 in the input space. A more accurate computation of the test dissimilarity measure *di,* which faithfully represents how dissimilar are the first latent representation *enc(IMGi)* and the second latent representation *enc(IMGi-R),* leads to a more accurate detection of anomalies, thus higher performance measures.

By computing the test dissimilarity measure *di* as disclosed, the invention overcomes the disadvantage that the computation of the Euclidean distance in the latent space depends on the parameterization, because the test dissimilarity measure *di* is invariant to parametrization. The advantage of the invention to use the test dissimilarity measure *di* having invariance to re-parametrization is that the computation of the test dissimilarity measure *di* does not depend on the way in which the second Variational AutoEncoder VAE model (VAE-U) represents the approximate posterior of said second Variational AutoEncoder VAE model (VAE-U).

By computing the test dissimilarity measure *di* as disclosed, the invention has the advantage to address all situations, irrespectively of whether the variability of the non-anomalous data is greater, equal or lower than the variability of the anomalous data, which allows a broader scope of industrial applicability of the invention.

The invention has the advantage that the detection of the anomalous data is more efficient since it computes a single test dissimilarity measure *di* as opposed to computing distances for multiple pairs of points as for example is the case in the Pereira *et al.* [8] approach.

In a preferred embodiment of the invention, the first dissimilarity measure *d-Hi* and the test dissimilarity measure di are computed using one among four methods of computation: the Kullback-Leibler divergence, the symmetrized Kullback-Leibler divergence, the Fisher-Rao distance or the Wasserstein distance. The preferred method of computation one among the four said methods is the same for the first dissimilarity measure *d-Hi* and the test dissimilarity measure *di.*

The Kullback-Leibler divergence, the symmetrized Kullback-Leibler divergence and the Fisher-Rao distance are invariant to the choice of the parameterization, which is an advantage as mentioned throughout the invention. For Gaussian distributions, the Wasserstein distance is a Riemannian distance and thus it is also invariant to the choice of parametrization. The Fisher-Rao distance, the symmetrized Kullback-Leibler divergence and the Wasserstein distance are symmetric with respect to their arguments, while the KL Kullback-Leibler is not. Moreover, the Kullback-Leibler KL divergence does not satisfy the triangle inequality. Consequently, if in a particular implementation of the invention it is important to take into account a dissimilarity measure that is symmetric with respect to its arguments, either the Fisher-Rao distance or the symmetrized Kullback-Leibler divergence or the Wasserstein distance shall be selected.

The Wasserstein distance takes into account the metric of the sample space in the computation of the distance, which is an advantage in case the impact of using different metrics in the sample space is considered. Indeed, the Wasserstein distance measures the work required to transport the probability density from one state to another one, such work being ignored by the Fisher-Rao distance, the Kullback-Leibler divergence, and the symmetrized Kullback-Leibler divergence. Consequently, if in another particular implementation of the invention it is important to take into account the work required to transport the probability density from one state to another one, the Wasserstein distance shall be selected for computation.

The Wasserstein distance does not require both measures - that is the two objects between the respective dissimilarity is computed, to be defined over the same probability space. In particular the Kullback-Leibler divergence may diverge if one of the two respective distributions corresponding to said objects has reduced support, in contrast with the Wasserstein distance which remain finite.

Concluding in respect to the choice of the method of computation of the first dissimilarity measure *d-Hi* and the test dissimilarity measure *di,* the possibility of choosing among the four said methods of computation has the advantage of flexibility, in order to adapt to the particulars of each particular implementation of the method. All four said methods of computation have the advantage that the manifold structure is taken into account in the computation of the dissimilarity measures.

In a preferred embodiment of the invention, the data case anomaly threshold *THRj* is computed either as a fixed, pre-determined percentile, whereas in another preferred embodiment of the invention, the data case anomaly threshold *THRj* is computed as a *P^{th}* percentile of the first plurality of dissimilarity measures *d-Hi* corresponding to the first plurality of data cases IMG-Hi of the validation sub-set of the first dataset H, where the *P^{th}* percentile is selected from a pre-determined list of percentiles *Pₙ* so as to maximize the F1 score on the validation sub-set of the second dataset U.

Using fixed, pre-determined percentile for the data case anomaly threshold *THRj* is particularly advantageous when there is a previous experience that the method of the invention is already optimized for the particular real-life situation in which it is used the respective fixed, pre-determined percentile.

Computation as the *P^{th}* percentile of the first plurality of dissimilarity measures is particularly advantageous when the method of the invention needs constant fine-tuning for the particular real-life situation in which it is applied.

The way the data case anomaly threshold *THRj* is computed can be combined with any of the four methods of computation of the first dissimilarity measure *d-Hi* and of the test dissimilarity measure *di* giving the advantage of flexibility of the method according to the invention to adapt the particular real-life situation in which it is applied.

In case of the preferred embodiment where the data case anomaly threshold *THRj* is computed as a *P^{th}* percentile, said computation of the data case anomaly threshold *THRj* is by selecting the *P^{th}* percentile from the pre-determined list of percentiles Pn. This is carried out in a separate set of training and validation sub-steps TR-PS as follows:
TR-PS-1 The computing module computes for each percentile Pn from the pre-determined list of percentiles a corresponding data case anomaly threshold *THRjn,*
TR-PS-2 The computing module carries out for each raw data case *IMG-Ui* from the validation sub-set of the second dataset U:
- determines a reconstruction of the data case *IMG-Ui* using the first Variational AutoEncoder VAE model (VAE-H), resulting a reconstructed data case *IMG-Ui-R,*
- encodes the data case *IMG-Ui* through the second Variational AutoEncoder model (VAE-U), resulting a first encoding *enc(IMG-Ui),*
- encodes the reconstructed data case *IMG-Ui-R* through the second Variational
AutoEncoder model (VAE-U), resulting a second encoding *enc(IMG-Ui-R),*
TR-PS-3 The computing module computes a second dissimilarity measure *d-Ui* between the first encoding *enc(IMG-Ui)* and the second encoding *enc(IMG-Ui-R),* resulting a second plurality of dissimilarity measures corresponding to the second plurality of data cases *IMG-Ui,*
TR-PS-4 The computing module compares said second plurality of the second dissimilarity measures *d-Ui* with the corresponding data case anomaly threshold *THRjₙ* for the respective percentile Pn,
TR-PS-5 The labelling module labels as an anomalous data case *IMG-Ui-Ran* of each data case *IMG-Ui-R* whose corresponding second dissimilarity measure *d-Ui* computed in TR-PS-4 is above the corresponding data case anomaly threshold *THRjₙ,*
TR-PS-6 The computing module computes the F1 score by use of the labels determined in TR-PS-5,
TR-PS-7 The labelling module selects the *P^{th}* percentile so as to maximize the F1 score on the validation sub-set of the second dataset U. The maximization of the F1 score is according to prior art.

The selected *P^{th}* percentile is used for the testing steps TS of the method for the particular real-life situation for which the method is intended. The method according to the invention is used in different fields to detect anomalous data, for example in the speech recognition to detect a particular sound, or in the automotive industry to detect a noise that indicates bad functioning of the engine or in medical imaging to detect the images corresponding to unhealthy subjects. In each of the afore-mentioned examples it is selected a different *P^{th}* percentile based on different pre-determined criteria. Once selected, said different *P^{th}* percentile may remain fixed or may change if the conditions of the real-life situations also change, for example:
- in the automotive industry, if the method is used to a new model of vehicle that has a completely different engine with completely different noise,
- in case of medical imaging, in case there is a new apparatus for providing raw images that provides images in a totally different format as compared with the new one or if there is a new medical condition for which it is necessary to detect the images corresponding to unhealthy subjects.

All the afore-mentioned examples are for illustrating the concept and advantage of using the selection of the *P^{th}* percentile to adjust to each particular real-life situation and not for limiting the scope of the invention to the afore-mentioned examples.

The computation of the second dissimilarity measure *d-Ui* in the above preferred embodiment where the data case anomaly threshold THRj is computed as the *P^{th}* percentile is done using one among the four methods of computation: the Kullback-Leibler divergence, the Fisher-Rao distance or the Wasserstein distance, namely the same as the one for the first dissimilarity measure *d-Hi* and for the test dissimilarity measure *di.*

The first dissimilarity measure *d-Hi,* the test dissimilarity measure *di* and the second dissimilarity measure *d-Ui* refer to the same function, however they are computed between different elements:
- the first dissimilarity measure *d-Hi* is computed in TR-S5 between the first encoding *enc(IMG-Hi)* and the second encoding *enc(IMG-Hi-R),*
- the test dissimilarity measure *di* is computed in TS-S3 between the first encoding *enc(IMGi)* and the second encoding enc*(IMGi-R)*,
- the second dissimilarity measure *d-Ui* is computed in TR-PS-3 between the first encoding *enc(IMG-Ui)* and the second encoding *enc(IMG-Ui-R).*

In another preferred embodiment the anomaly is localized in the at least one anomalous data case *IMG-An* by carrying out of the following sub-steps of sub-step TS-S6:
TS-S6-1 The computing module computes the geodetic γ between the first encoding *enc(IMGi)* and second encoding *enc(IMGi-R)* such that γ(0) = *enc(IMGi)* and γ(1) = *enc(IMGi-R)*,
TS-S6-2 The computing module generates, using the second Variational AutoEncoder VAE model (VAE-U), a plurality of reconstructed data cases *IMG1-R, IMG2-R, ....IMGk-R* obtained by decoding γ(t1), γ(t2),..., γ(tk) for different values t1, t2, ..., tk, where t1, t2, ..., tk are real numbers in the interval [0,1], equally spaced or not equally spaced, corresponding to the plurality of positions along the geodetic γ, and where γ(t1), γ(t2),..., γ(tk) are points in the latent space of the second Variational AutoEncoder VAE model (VAE-U) on the curve between the first encoding *enc(IMGi)* and the second encoding *enc(IMGi-R),*
TS-S6-3 The computing module computes the measure of variability *Var(p)* for each data point *p* of the plurality of reconstructed data cases *IMG1-R, IMG2-R, ....IMGk-R,*
TS-S6-4 The computing module compares said measure of variability *Var(p)* with a data point pre-defined anomaly threshold *THRp.* The data point pre-defined anomaly threshold *THRp* is a percentile of the distribution of the value of said measure of variability *Var(p)* for the data points *p* computed for the first plurality of data cases *IMG-Hi* of the validation sub-set of the first dataset H.

The selection of the data point pre-defined anomaly threshold *THRp* is similar to the selection of the data case anomaly threshold THRj, for the cases when said data case anomaly threshold *THRj* is computed as a fixed, pre-determined percentile. The percentile of the distribution of the variability measure *Var(p)* is considered for all data points p and all data cases *IMGi.*

TS-S6-5 The labelling module labels each data point *p* in the anomalous data case IMG-An as an anomalous data point *pa*n if the corresponding measure of variability Var(p) is above the data point pre-defined anomaly threshold *THRp.*

In other words, in this preferred embodiment, the anomaly mask is determined in TS S6 by means of computing the measure of variability *Var(p)* for each data point *p* of the reconstructed data cases *IMG1-R, IMG2-R, ....IMGk -R* obtained by computing geodetics in the latent space of the second Variational AutoEncoder model (VAE-U).

One non-limiting example of the measure of variability *Var(p)* is the variance. Another non-limiting example of the measure of variability *Var(p)* is the standard deviation.

In other preferred embodiments of the invention, the data cases of the first dataset H, of the second dataset U and of the test dataset of any of the above described preferred embodiments are images and the data points *p* are pixels *p.*

A particular case of the preferred embodiments when the data cases are images is when the images are used in medical imaging. In this case, the first dataset H containing the first plurality of images *IMG-Hi* without anomalies corresponds to healthy people, whereas the second dataset U containing the second plurality of images *IMG-Ui* as a mix of unlabelled images with and without anomalies, corresponds to unhealthy and, respectively, healthy people.

When the data cases are images used in medical imaging, there are several adaptations:
- the pre-processing carried out in TR-S2 and TS-S1 may include histogram matching and bias field correction,
- the anomaly mask is determined by means of computing the measure of variability *Var(p)*for each pixel *p* of the reconstructed images obtained by computing geodetics in the latent space of the second Variational AutoEncoder model (VAE-U). The result of the ensemble of steps TS-S6-1 to TS-S6-5 for the embodiments where the data cases are images used in medical imaging is the anomaly mask shown in Fig. 4).

This particular case of images used in medical imaging is described in detail in the example of realization.

In other preferred embodiments of the invention the data cases are sounds. In this case, the encoders and decoders of the first Variational AutoEncoder (VAE-H) and, respectively of the second Variational AutoEncoder (VAE-U) are configured to capture the time dimension of the signal, for instance by using 1D convolutional filters, recurrent neural networks or more in general autoregressive components. An example of decoders used in AutoEncoders for audio signals is disclosed in WO2018/048934. Provided the first Variational AutoEncoder (VAE-H) and, respectively the second Variational AutoEncoder (VAE-U) are able to learn meaningful hidden representations of audio signals, the reconstruction of the audio signal would work in a similar way with the reconstruction of the images. The comparison of the latent representation by computing in the latent space the first dissimilarity measure *d-Hi,* the test dissimilarity measure *di* and the second dissimilarity measure *d-Ui* is adapted to take into account the time dimension of the sound, said adaptations being done, for example, by defining alternative distances functions between latent representation which are invariant to a time shift of the sound signal.

In a **second** aspect of the invention it is provided a machine computing unit comprising at least one processor, at least one memory, a data collecting and pre-processing module, a computing module, a labelling module, and a communication module, the machine computing unit being configured for detecting anomalous data in a test dataset comprising at least one raw data case *IMGi* by means of carrying out the computer-implementing method of the first aspect of the invention in any of its embodiments and combinations thereof.

The machine computing unit is a single computing unit or a plurality of computing units located remotely from one another communicating within a computer communication system.

For the sake of simplicity in the invention, it shall be understood that:
- the storage of the instant values of all variables disclosed in the invention is carried out according to prior art in one or more non-volatile memory units of the machine computing unit,
- the data collecting and pre-processing module, the computing module, the labelling module, and the communication module are configured to communicate between themselves in such a way to allow the data corresponding to each step of the method of the first aspect of the invention in any of its embodiments and combinations thereof to be transmitted from one module to another,
- the machine computing unit is configured to communicate with the at least one apparatus providing raw data cases *IMGi,* for the purpose of receiving the raw data cases *IMGi,* the communication and storage of the raw data cases *IMGi* taking place according to prior art.

A non-limiting example of machine computing unit is a computer.

In a **third** aspect of the invention it is provided a computer program comprising instructions which, when the program is executed by the machine computing unit, cause the machine computing unit to carry out the computer-implementing method of the first aspect of the invention in any of its embodiments and combinations thereof.

In a **fourth** aspect of the invention is provided a computer readable medium having stored thereon instructions which, when executed by the machine computing unit, cause the machine computing unit to carry out the computer-implementing method of the first aspect of the invention in any of its embodiments and combinations thereof.

In a **fifth** aspect of the invention is provided a data stream which is representative of the computer program of the invention.

### Example of realization

An example of realization is provided for a better understanding of the invention and not for limiting it.

The field of the example is medical imaging. Thus, data cases are images. In particular, the example refers to brain Magnetic Resonance Images MRIs. The non-anomalous data cases are images of healthy brains, whereas the anomalous data cases are images of brains that have one or more tumours. The purpose of the method is thus to classify the images of a subject as outputted by the apparatus carrying out Magnetic Resonance Images MRIs into either normal or anomalous and, in case of images of anomaly to localize the anomaly on the image, as this corresponds to localizing the tumour or tumours in the brain of the subject.

When using brain images, the pre-processing of said images before starting the method according to the invention must include removing from the images the bones of the skull because only the tissue of the brain is subject to analysis.

The first dataset H selected in TR-S1 is the publicly available dataset HCP [9] containing the first plurality of data cases *IMG-Hi* without anomalies, corresponding to healthy brains.

The second dataset U selected in TR-S1 is the publicly available dataset BRATS 2018 [10,11], containing the second plurality of data cases *IMG-Ui* as a mix of unlabelled data with and without anomalies, corresponding to brains with tumours, respectively brains without tumours.

The HCP dataset represents a mixture of images gathered from 1,200 healthy subjects. In the example of the realization, it was used one of the sub-sets available for the HCP dataset, which contains 214 T2-weighted Magnetic Resonance Images MRIs scans of unrelated subjects. For each scan representing a 3D image, 190 slices depicting brain tissue were kept, a slice being a 2D-image.

In this case:
- the first dataset H training sub-set is the training sub-set of the HCP dataset; it contains 149 scans, and 149 scans * 190 slices =28,310 2D images corresponding to 149 subjects,
- the first dataset H validation sub-set is the validation sub-set of the HCP dataset; it contains 32 scans, and 32 scans * 190 slices = 6,080 2D images corresponding to 32 subjects and
- the first dataset H test sub-set is the testing sub-set of the HCP dataset it contains 33 scans, and 33 scans * 190 slices = 6,270 2D images corresponding to 33 subjects; This testing sub-set of the HCP dataset will be used in the testing steps TS.

Thus, the first plurality of data cases *IMG-Hi* when the first dataset H is the HCP dataset has 28,310+6,080+6,270=40,660 2D-images.

The BRATS 2018 dataset comprises a mix of pre-therapy and post-therapy multi-contrast Magnetic Resonance Images MRIs scans from glioma subjects. The inventors had only access to the initial training sub-set of the BRATS 2018 dataset, which was split into training, validation and testing sub-set as follows, knowing that the middle 130 2D slices from each scan were kept:
- the second dataset U training sub-set is the training sub-set resulting from the split of the initial training sub-set of the BRATS 2018 dataset; it contains 199 scans, and 199 scans * 130 slices = 25,870 2D-images corresponding to 199 subjects,
- the second dataset U validation sub-set is the validation sub-set resulting from the split of the initial training sub-set of the BRATS 2018 dataset; it contains 42 scans, and 42 scans * 130 slices 5,460 2D- images corresponding to 42 subjects,
- the second dataset U test sub-set is the testing sub-set resulting from the split of the initial training sub-set of the BRATS 20185 dataset; it contains 44 scans, and 44 scans * 130 slices = 5,720 2D images corresponding to 44 subjects. This testing sub-set will be used in the testing steps TS.

The second plurality of data cases *IMG-Ui* when the second dataset U is the BRATS 2018 dataset has 25,870+5,460+5,720=37,050 2D-images.

In TR-S2, the first plurality of data cases *IMG-Hi* and the second plurality of data *cases IMG-Ui* were cropped and resized to 200 x 200 and down-sampled to 128 X 128 pixels. To avoid overfitting during the training and validation steps TR, the left and right hemispheres from the slices have been flipped with probability 0.5. Pre-processing included bias field correction, histogram matching, data augmentation such as adjustment of brightness and injection of Gaussian white noise with standard deviation equal to 0.01. The data augmentation technique has proved to be useful for enhancing generalization from the training to the validation.

The first Variational AutoEncoder VAE model (VAE-H) and the second Variational AutoEncoder VAE model (VAE-U) have the following network architectures and training parameters:
- The encoders are convolutional neural networks with channels [64, 128, 256, 512] for both models, kernel size 4 × 4 and strides 2, followed by a stochastic layer of independent Gaussian distributions with size 128,
- The decoders have output channels [512, 256, 128, 64], kernel size 4 × 4 and strides 2, followed by a logit-normal distribution with a clip value for the mean equal to 0.01 and 0.001 respectively and a covariance (scalar for HCP and vectorial for BRATS) with a minimum value of 0.01,

- For each input, 3 samples are generated in the latent space during training,
- The models have been trained with Adam for 100 epochs, learning rate 0.0001, and default values for the β parameters,
- Batch size has been set to 32,
- The global norm of the gradient has been clipped to 1,000 to avoid numerical instabilities.

The algorithm for the classification of brain Magnetic Resonance Image MRI is as follows:

| | |
|---|---|
| Input: | Let *di* be the test dissimilarity measure over the latent space of the second Variational AutoEncoder model (VAE-U) |
| Data: | First dataset H is the HCP dataset of Magnetic Resonance Image MRI of healthy subjects containing a first plurality of images *IMG-Hi* |
| | Second dataset U is the BRATS-2018 dataset of Magnetic Resonance Image MRI that may contain tumoral tissues containing a second plurality of images *IMG-Ui* |
| 1 | Let the first Variational AutoEncoder VAE model (VAE-H) be trained on the HCP dataset |
| 2 | Let second Variational AutoEncoder VAE model (VAE-U) be trained on the BRATS-2018 dataset |
| 3 | Let *x* be an image from the second plurality of images *IMG-Ui* |
| 4 | Let *xh* be the reconstruction *IMG-Ui-R1* of the image *x* using the first Variational AutoEncoder VAE model (VAE-H), |
| 5 | Let *VAE.enc (x)* be the first encoding *enc(IMGi*), that is the encoding of the image x through the second Variational AutoEncoder model (VAE-U) |
| 6 | Let *VAE enc (xh)* be the second encoding *enc(IMGi-R*), that is the encoding of the reconstruction xh through the second Variational AutoEncoder model (VAE-U) |
| 7 | Compute the distribution of the plurality of the test dissimilarity measure *di(x)* with *x* in the validation sub-set of the HCP dataset as the first dataset H and let d* be the data case anomaly threshold *THRj* based on a fixed percentile |
| 8 | Compute *di(x)* with *x* taking values in the test sub-set of the BRATS-2018 dataset as the second dataset U |
| 9 | Label the slice as normal if *di(x) < d** |

The test dissimilarity measure *di* chosen is the Wasserstein distance between the approximate posteriors obtained by encoding *x* and *xh* through the second Variational AutoEncoder model (VAE-U).

After training and validation of the first Variational AutoEncoder VAE model (VAE-H) and of the second Variational AutoEncoder model (VAE-U), the distribution of *d(x)* was computed for x taking values in the validation sub-set of the BRATS-2018 dataset as the second dataset U. This allowed to fix a value for the d*, the data case anomaly threshold THRj, based on a 99% percentile.

Fig. 5) schematically illustrates the reconstructions *xh,* through VAE-H as part of the TS-S2 sub-step:
- First row - raw images *IMGi* from the test sub-set of the BRATS-2018 dataset,
- Second row- reconstructed images *IMG-Ui-R* using the first Variational AutoEncoder VAE model (VAE-H),

Fig. 6) illustrates a histogram of the test dissimilarity measures *di* in the space of the approximate posteriors of the second Variational AutoEncoder model (VAE-U) between *VAE.enc (x)* and *VAE enc (xh).* The vertical line corresponds to the data case anomaly threshold *THRj* based on a 99% fixed percentile computed for the validation sub-set of the HCP dataset. The threshold *d** is used to label the images from the test sub-set of the BRATS-2018 dataset based on the value of *di(x).*

Since not all the slices of the subjects of the test sub-set of the BRATS-2018 dataset contain tumoral tissues, in Fig. 6) the test sub-set of the BRATS-2018 dataset has been split into two categories: images of healthy subjects, corresponding to normal data and images of subject with tumoral tissues, corresponding to anomalous data.

To check if the method meets at least one of the performance measures mentioned the section "problem to be solved by the invention" a comparison is made between the method of the invention and the method of anomaly detection of prior art computing dissimilarities in the input space:
- the L2 in the input space between the original raw image *IMGi* and its reconstruction *IMGi -Ui-R* using the first Variational AutoEncoder VAE model (VAE-H), and

Fig.7) shows the histogram of the L2 distance in the input space between the image x and its reconstruction xh using the first Variational AutoEncoder VAE model (VAE-H) for x taking values in the test sub-set of the BRATS-2018 dataset as the second dataset U. The distribution of the L2 in the input space shows a lower degree of separation between healthy and unhealthy slices of the test sub-set of the BRATS-2018 dataset as compared with the histogram of the test dissimilarity measures *di* in the space of the approximate posteriors of the second Variational AutoEncoder model (VAE-U) shown in Fig. 6). Lower degree of separation means that the threshold between healthy and unhealthy slices is able to separate correctly a smaller number of slices in the example of prior art as compared with the method of the invention, thus the invention provides a higher performance of the anomaly detection for the test data cases.

Table 1 shows the computation of the accuracy, F1 score and Area under the Receiver operating characteristic curve ROC AUC for the test sub-set of the BRATS-2018 dataset for the example of realization when the test dissimilarity measure *di* is chosen as the Wasserstein distance between the approximate posteriors obtained by encoding *x* and xh through the second Variational AutoEncoder model (VAE-U). For the computation of ROC AUC, the anomaly scores are given by the values of the test dissimilarity measure *di.* For the accuracy and F1 score, the predictions are computed according to the algorithm previously described.

The images with a tumour area of above 20 pixels in the original images before the resizing are labelled as anomalous. The value 20 pixels is set due to the fact that resizing the images makes small tumours, in this case, tumours with are less than 20 pixels difficult to detect. The value of 20 has been suggested by the studies of Zimmerer, D. et al.[12].

**Table 1- higher performance of the anomaly detection of the invention using all three performance measures.**

| Method | Accuracy | F1 score | AUC |
|---|---|---|---|
| L2 input space | 0.70 | 0.54 | 0.85 |
| Method according to the invention | 0.80 | 0.78 | 0.88 |

As it can be seen, the method according to the invention has better scores for each of the three performance measures: accuracy, F1 score and Area under the Receiver operating characteristic curve ROC AUC, as compared with the method of prior art of computing the L2 in the input space.

While the description of the method and the system was disclosed in detail in connection to preferred embodiments, those skilled in the art will appreciate that changes may be made to adapt a particular situation without departing from the essential scope to the teaching of the invention. Such changes can be made without departing from the spirit and scope of the invention and without diminishing its advantages. It is therefore intended that such changes be covered by the appended claims.

## Claims

1. A computer-implemented method for detecting anomalous data in a test dataset comprising at least one raw data case *IMGi* using two Variational AutoEncoders VAEs, by means of using a machine-computing unit, the method comprising:
**TRAINING AND VALIDATION STEPS:**
TR-S1 Collecting simultaneously, by means of a data collecting and pre-processing module, two datasets:
- a first dataset H containing a first plurality of data cases *IMG-Hi* without anomalies, corresponding to non-anomalous data cases, said first plurality of data cases *IMG-Hi* belonging to a training sub-set of the first dataset H and to a validation sub-set of the first dataset H,
- a second dataset U containing a second plurality of data cases *IMG-Ui* as a mix of unlabelled data with and without anomalies, corresponding to anomalous data cases and, respectively, non-anomalous data cases, said second plurality of data cases *IMG-Ui* belonging to a training sub-set of the second dataset and to a validation-sub-set of the second dataset U.
TR-S2 Pre-processing by means of the data collecting and pre-processing module said first dataset H and said second dataset U,
TR-S3 Training and validation simultaneously by means of a computing module of:
- a first Variational AutoEncoder VAE model (VAE-H) on the first plurality of data cases *IMG-Hi* such that it learns representations of non-anomalous data cases,
- a second Variational AutoEncoder VAE model (VAE-U) on the second plurality of data cases *IMG-Ui* such that it learns variegated representations of data cases with and without anomalies.
TR-S4 Carrying out, by means of the computing module, for each data case *IMG-Hi* of the validation sub-set of the first dataset H:
- determining a reconstruction of the data case *IMG-Hi* using the first Variational AutoEncoder VAE model (VAE-H), resulting the reconstructed data case *IMG-Hi-R,*
- encoding the data case *IMG-Hi* through the second Variational AutoEncoder model (VAE-U), resulting a first encoding *enc(IMG-Hi),*
- encoding the reconstructed data case *IMG-Hi-R* through the second Variational AutoEncoder model (VAE-U), resulting a second encoding *enc(IMG-Hi-R),*
TR-S5 Computing, by means of the computing module, of a first dissimilarity measure *d-Hi* between the first encoding *enc(IMG-Hi)* and the second encoding *enc(IMG-Hi-R),* resulting a first plurality of dissimilarity measures corresponding to the first plurality of data cases *IMG-Hi,*
TR-S6 Computing, by means of the computing module, of a data case anomaly threshold *THRj* based on the first plurality of dissimilarity measures computed for the first dataset H,
**TESTING STEPS** for detecting anomalous data in the test dataset comprising the at least one raw data case *IMGi,* said at least one raw data case IMGi comprising at least one data point *p*,
TS-S1 Receiving, by means of the collecting and pre-processing module, of the at least one raw data case *IMGi* from at least one apparatus providing raw data cases *IMGi* and pre-processing the at least one raw data case *IMGi,*
TS-S2 Carrying out, by means of the computing module,
- determining a reconstruction of the at least one raw data case *IMGi* using the first Variational AutoEncoder VAE model (VAE-H), resulting the reconstructed data case *IMGi-R,*
- encoding the at least one raw data case *IMGi* through the second Variational AutoEncoder model (VAE-U), resulting a first encoding *enc(IMGi)*,
- encoding the reconstructed data case *IMGi-R* through the second Variational AutoEncoder model (VAE-U), resulting a second encoding *enc(IMGi-R),*
TS-S3 Computing, by means of the computing module, of a test dissimilarity measure *di* between the first encoding *enc(IMGi)* and the second encoding *enc(IMGi-R),*
TS-S4 Comparing, by means of the computing module, of said test dissimilarity measure *di* with the data case anomaly threshold *THRj*,
TS-S5 Labelling, by means of a labelling module, of the at least one raw data case *IMGi* as an anomalous data case *IMG-An* if the corresponding test dissimilarity measure *di* computed in TS-S3 is above said data case anomaly threshold THRj,
TS-S6 For the at least one anomalous data case *IMG-An* labelled in TS-S5, localizing, by means of the labelling module, of the anomaly in the at least one anomalous data case *IMG-An,* by determining an anomaly mask of the at least one data point *p,*
TS-S7 Sending, by means of the labelling module, of the anomalous data case *IMG-An* together with the corresponding localization of the anomaly to a communication module accessible to a decision maker.

2. The computer-implemented method of any claim 1, *wherein* the first dissimilarity measure *d-Hi* and the test dissimilarity measure *di* are computed using one among four methods of computation: the Kullback-Leibler divergence, the Fisher-Rao distance or the Wasserstein distance, and wherein said one among four methods of computation is the same for the first dissimilarity measure *d-Hi* and the test dissimilarity measure *di.*

3. The computer-implemented method of claim 1 or 2, *wherein* the data case anomaly threshold THRj is computed as a fixed, pre-determined percentile.

4. The computer-implemented method of claims 1 or 2, wherein the data case anomaly threshold THRj is computed as a *P^{th}* percentile of the first plurality of dissimilarity measures *d-Hi* corresponding to the first plurality of data cases *IMG-Hi* of the first dataset H, and
wherein the *P^{th}* percentile is selected from a pre-determined list of percentiles *Pₙ* so as to maximize the F1 score on the validation sub-set of the second dataset U.

5. The computer-implemented method of claim 4, wherein the sub-steps for selecting the *P^{th}* percentile from the pre-determined list of percentiles *P^{th}ₙ* are determined as follows:
TR-PS-1 Computing, by means of the computing module, for each percentile Pn from the pre-determined list of percentiles a corresponding data case anomaly threshold *THRjₙ,*
TR-PS-2 Carrying out, by means of the computing module, for each raw data case *IMG-Ui* of the validation sub-set of the second dataset U:
- determining a reconstruction of the data case *IMG-Ui* using the first Variational AutoEncoder VAE model (VAE-H), resulting a reconstructed data case *IMG-Ui-R,*
- encoding the data case *IMG-Ui* through the second Variational AutoEncoder model (VAE-U), resulting a first encoding *enc(IMG-Ui),*
- encoding the reconstructed data case *IMG-Ui-R* through the second Variational AutoEncoder model (VAE-U), resulting a second encoding *enc(IMG-Ui-R),*
TR-PS-3 Computing, by means of the computing module, of a second dissimilarity measure *d-Ui* between the first encoding *enc(IMG-Ui)* and the second encoding *enc(IMG-Ui-R),* resulting a plurality of second dissimilarity measures corresponding to the second plurality of data cases *IMG-Ui,*
TR-PS-4 Comparing, by means of the computing module, said second plurality of the second dissimilarity measures *d-Ui* with the corresponding data case anomaly threshold *THRjₙ* for the respective percentile *Pₙ,*
TR-PS-5 Labelling, by means of the labelling module, as an anomalous data case *IMG-Ui-Ran* of each data case *IMG-Ui-R* whose corresponding second dissimilarity measure *d-Ui* computed in TR-PS-4 is above the corresponding data case anomaly threshold *THRjₙ,*
TR-PS-6 Computing, by means of the computing module, the F1 score by use of the labels determined in TR-PS-5,
TR-PS-7 Selecting, by means of the labelling module, the P^{th} percentile so as to maximize the F1 score on the validation sub-set of the second dataset U.

6. The computer-implemented method of claim 5, *wherein* the second dissimilarity measure *d-Ui* is computed using one among the four methods of computation: the Kullback-Leibler divergence, the Fisher-Rao distance, the symmetrized Kullback-Leibler divergence, or the Wasserstein distance, and wherein said one among the four methods of computation is the same as the one for the first dissimilarity measure *d-Hi* and for the test dissimilarity measure *di.*

7. The computer-implemented method of any preceding claim, *wherein* the anomaly is localized in the at least one anomalous data case *IMG-An* by carrying out of the following sub-steps of sub-step TS-S6:
TS-S6-1 Computing, by means of the computing module, the geodetic γ between the first encoding *enc(IMGi)* and second encoding *enc(IMGi-R)* such that γ(0) = *enc(IMGi)* and γ(1) = *enc(IMGi-R)*,
TS-S6-2 Generating, by means of the computing module, using the second Variational AutoEncoder VAE model (VAE-U), of a plurality of reconstructed data cases *IMG1-R, IMG2-R,* ....*IMGk-R* obtained by decoding γ(t1), γ(t2),..., γ(tk) for different values t1, t2, ..., tk,
where t1, t2, ..., tk are real numbers in the interval [0,1], corresponding to the plurality of positions along the geodetic γ, and where γ(t1), γ(t2),..., γ(tk) are points in the latent space of the second Variational AutoEncoder VAE model (VAE-U) on the curve between first encoding *enc(IMGi)* and second encoding *enc(IMGi-R),*
TS-S6-3 Computing, by means of the computing module, the measure of variability *Var(p)* for each data point *p* of the plurality of reconstructed data cases *IMG1, IMG2,* ....*IMGk*.
TS-S6-4 Comparing, by means of the computing module, of said measure of variability *Var(p)* with a data point pre-defined anomaly threshold *THRp*, said data point pre-defined anomaly threshold *THRp* computed as a percentile of the distribution of the value of said measure of variability *Var(p)* for the data points *p* computed for the first plurality of data cases IMG-Hi of the validation sub-set of the first dataset H,
TS-S6-5 Labelling, by means of the labelling module, of each data point *p* in the anomalous data case *IMG-An* as an anomalous data point *pa*n if the corresponding measure of variability *Var(p)* is above the data point pre-defined anomaly threshold *THRp.*

8. The computer-implemented method of any preceding claim, *wherein* the first plurality of data cases of the first dataset H, of the second dataset U and of the test dataset are images, and *wherein* the data points *p* are pixels *p.*

9. The computer-implemented method of claim 8, *wherein* the images are used in medical imaging, the first dataset H containing the first plurality of images *IMG-Hi* without anomalies, corresponding to healthy people, the second dataset U containing the second plurality of images *IMG-Ui* as a mix of unlabelled images with and without anomalies, corresponding to unhealthy and, respectively, healthy people.

10. A machine computing unit comprising at least one processor, at least one memory, a data collecting and pre-processing module, a computing module, a labelling module, and a communication module, the machine computing unit being configured for detecting anomalous data in a test dataset comprising at least one raw data case *IMGi* by means of carrying out the computer-implementing method of any of the claims from 1 to 9.

11. Computer program comprising instructions which, when the program is executed by the machine computing unit according to claim 10, cause the machine computing unit to carry out the steps of the method, according to any of the claims from 1 to 9.

12. A computer readable medium having stored thereon instructions which, when executed by the machine computing unit according to claim 11, cause the machine computing unit to carry out all the steps of the method, according to any of the claims from 1 to 9.

13. A data stream which is representative of a computer program according to claim 11.
